## Europäisches Patentamt
## European Patent Office
## Office européen des brevets

(11) Numéro de publication: **0 227 594**
**B1**

(12) **FASCICULE DE BREVET EUROPEEN**

(45) Date de publication du fascicule du brevet:
**27.09.89**

(21) Numéro de dépôt: **86810542.0**

(22) Date de dépôt: **26.11.86**

(51) Int. Cl.⁴: **A61B 17/60**

(54) Dispositif de positionnement et de blocage d'une pièce présentant des zones ciculaires.

(30) Priorité: **29.11.85 CH 5119/85**
**29.09.86 CH 3894/86**

(43) Date de publication de la demande:
**01.07.87 Bulletin 87/27**

(45) Mention de la délivrance du brevet:
**27.09.89 Bulletin 89/39**

(84) Etats contractants désignés:
**AT BE CH DE ES FR GB GR IT LI LU NL SE**

(56) Documents cités:
**WO-A-83/02554**
**FR-A- 2 439 002**
**FR-A- 2 517 535**

(73) Titulaire: **JAQUET ORTHOPEDIE S.A., 5, chemin des Aulx, CH-1228 Plan-les-Ouates(CH)**

(72) Inventeur: **Wagenknecht, Marcel, 12, chemin des Milans, CH-1219 Le Lignon(CH)**

(74) Mandataire: **Dietlin, Henri, Dietlin & Cie S.A. Rue des Epinettes 19, CH-1227 Genève(CH)**

## Description

La présente invention concerne un dispositif de fixation permettant d'orienter la pièce maintenue, qui est plus particulièrement utilisé dans le domaine des appareils de fixation externe d'os.

En effet, pour la réduction d'os fracturés par exemple, on a développé depuis longtemps une technique de fixation de broches dans les os, broches qui sont soit prolongées dans l'os voisin, soit reprises dans un fixateur externe comportant une armature, afin d'assurer le maintien des éléments fracturés.

On connaît déjà des armatures comprenant des arceaux, telles que décrites dans le brevet CH 630.798.

On a étudié également la forme de la section de cet arceau, pour préférer une forme décrite dans le brevet suisse N° 664 079, forme permettant de créer des possibilités multiples et étendues de montage de broches ainsi que de tiges d'armatures ou autres pièces ou organes de liaison.

Les éléments constitutifs de ces fixateurs externes doivent être étudiés dans un double but. Tout d'abord ils doivent être aussi simples que possible, pour faciliter le travail du médecin au cours de l'opération, tout en autorisant une large gamme de positionnements éventuels. D'autre part, ils doivent permettre une fixation parfaitement rigide et sûre, pour éviter que la réduction de la fracture ne soit compromise au cours de la convalescence.

On connaît des dispositifs de positionnement et de blocage comprenant des mâchoires enserrant une pièce, les surfaces des mâchoires et de la pièce à enserrer en contact étant réalisées de manière à présenter une certaine rugosité obtenue par exemple par sablage. Ces dispositifs donnent habituellement satisfaction, mais avec le temps les surfaces rugueuses s'émoussent et la rugosité disparaît. Au fur et à mesure que les surfaces en contact deviennent plus lisses, le blocage devient de moins en moins efficace.

On a également proposé des dispositifs de blocage comprenant des surfaces planes présentant des rainures. Ces surfaces planes enserrent habituellement un cylindre ou une sphère en matière plastique. En variante les rainures peuvent également être appliquées sur la pièce présentant une partie arrondie à enserrer. Un tel dispositif est par exemple décrit dans la demande de brevet PCT WO 83/02554. Ce dispositif donne de bons résultats, mais la pièce en plastique subit rapidement de détériorations de sorte que sa durée de vie est relativement courte.

Le but de la présente invention est de supprimer ces inconvénients et de présenter un dispositif de positionnement et blocage et présentant une durée de vie relativement longue.

Le dispositif de positionnement et de blocage selon l'invention comprend deux mâchoires agencées pour être pressées l'une contre l'autre et enserrer la pièce, la pièce et/ou les mâchoires comprenant des zones circulaires présentant une succession de facettes régulièrement disposées sur les zones et destinées à coopérer avec la pièce ou la mâchoire complémentaire.

Il est caractérisé en ce que les facettes (81) sont constituées par des polygones réguliers et en ce que lesdites facettes adjacentes sont planes ou incurvées et forment entre elles des arêtes vives ou des pointes qui sont aptes à créer des déformations élastiques dans la pièce ou la mâchoire complémentaire, afin de réaliser un blocage efficace.

Cette pièce comporte également un alésage central destiné à recevoir une tige ou une fiche. Elle peut être divisée en deux parties selon l'axe de l'alésage central ou faire partie intégrante d'un tube prolongeant la sphère, soit d'un seul côté, soit de part et d'autre de celle-ci.

De plus la pièce à enserrer peut se présenter sous forme d'un cylindre ou d'un tronc de cône comprenant au moins un segment ou zone muni de facettes. Le cylindre à enserrer peut également présenter un alésage central perpendiculaire à son axe destiné à recevoir une fiche ou une tige et être divisé en deux parties selon l'axe de l'alésage central.

La succession de facettes est disposée sur un matériau plus dur que le matériau des mâchoires enserrant les zones à facettes. Ces matériaux peuvent être respectivement de l'acier trempé et de l'acier non trempé, ou de l'acier et un métal léger, ou des alliages en métal léger de duretés différentes, ou des alliages et des matières plastiques.

Selon un mode d'exécution préférentiel, les mâchoires enserrant la pièce présentant une partie arrondie sont serrées par deux vis dont les axes forment un angle l'un par rapport à l'autre, de sorte que par serrage l'un au moins des éléments fléchit pour assurer un meilleur blocage de la partie arrondie de la pièce.

Le dessin annexé représente, à titre d'exemples, plusieurs modes d'exécution ainsi que des variantes d'un dispositif de positionnement et de blocage d'une pièce selon l'invention.

Dans le dessin :

la figure 1 est une vue en perspective d'un dispositif de positionnement et de blocage destiné à être placé sur un élément d'arceau circulaire (représenté en traitillés) et maintenant une rotule de guidage (également en traitillés au dessin) comportant une partie tubulaire partant de la sphère centrale;

la figure 2 est une vue éclatée des constituants de la figure 1;

la figure 3 est une vue latérale du dispositif représenté auparavant, présentant ici une rotule avec une partie tubulaire s'étendant de part et d'autre de la sphère centrale;

la figure 4 est une vue en coupe, selon IV-IV, du dispositif de la figure 3;

la figure 5 est une vue de côté d'une rotule sphérique;

les figures 6A et 6B sont deux variantes de la rotule de la figure 5, vues en coupe;

la figure 7 est une vue latérale d'une variante de rotule;

la figure 8 est une vue agrandie de la partie centrale de la rotule de la figure 3, montrant des facettes régulièrement disposées sur le pourtour de la partie sphérique;

la figure 9 est une vue encore agrandie d'une petite partie de la surface de la rotule présentée à la figure 8, montrant le détail des facettes, dans une première forme d'exécution montrant des facettes planes;

la figure 10 est une vue en coupe selon X-X à la figure 9, présentant encore une partie de la mâchoire;

la figure 11 est une vue en coupe selon XI-XI à la figure 9, également avec une partie de la mâchoire;

la figure 12 est similaire à la figure 10, dans une seconde forme d'exécution présentant des facettes concaves;

la figure 13 est une vue en perspective d'un dispositif de positionnement et de blocage destiné à recevoir des fiches transfixiantes;

la figure 14 est une vue latérale, avec coupe partielle de la partie supérieure de la mâchoire de la figure 13, partie destinée à positionner et à bloquer une barre de liaison, et

la figure 15 est une vue latérale d'une variante de la partie représentée à la figure 14.

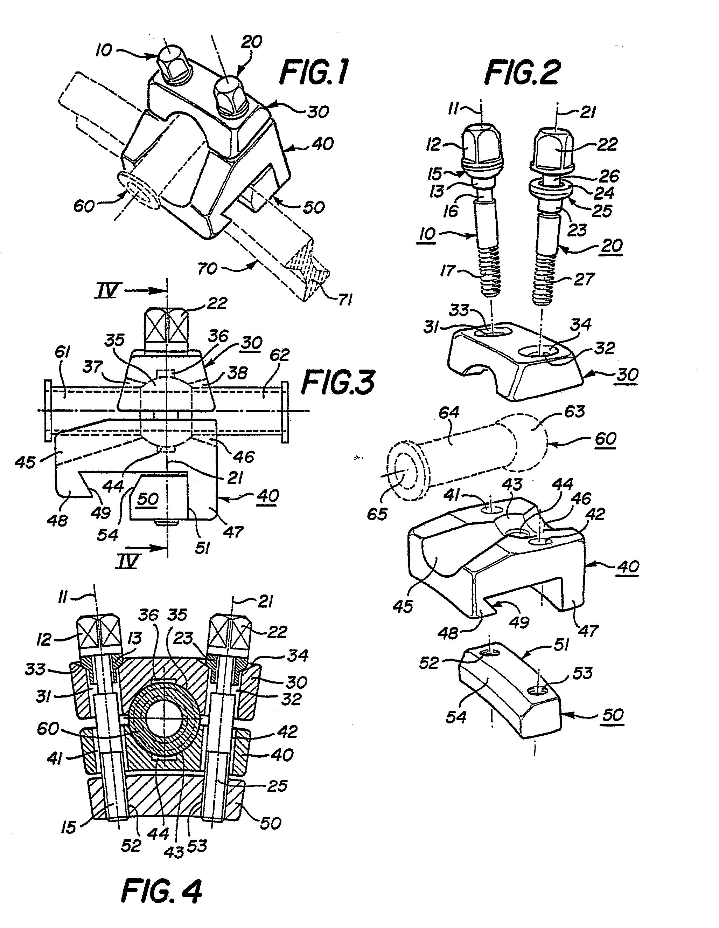

Le dispositif de positionnement et de blocage représenté aux figures 1 à 4 comporte principalement deux vis de fixation 10 et 20, destinées à traverser un élément supérieur 30 et un élément inférieur 40, avant de venir coopérer avec une contre-pièce 50, tout en permettant le positionnement et la fixation d'une rotule 60 ainsi que le serrage de l'ensemble sur un arceau 70.

Il faut tout d'abord noter que des axes 11 et 21 des vis de fixation 10 et 20 ne sont pas parallèles, mais forment un angle d'environ 10° pour une raison qui sera explicitée par la suite.

Les vis 10 et 20 présentent de larges têtes 12 et 22, pour faciliter leur mise en place et leur serrage manuel, avant d'être bloquées au moyen de tout outil adéquat lorsque le système est en place. Les vis représentées ont des têtes 12 et 22 carrées, mais il va sans dire que toute autre forme de tête est envisageable sans sortir du cadre de la présente invention.

En outre, les vis 10 et 20 comportent des rondelles 13 et 23 présentant chacune une face plane 14, 24 destinée à s'appuyer sur la tête de vis correspondante 12 cu 22, ainsi qu'une partie sphérique 15, 25, sur sa face opposée, dont le besoin sera énoncé par la suite. Il est à noter que les rondelles 13 et 23 sont engagées une fois pour toutes sur les vis 10 et 20, puis serties à leur base dans des rétrécissements 16 et 26 des vis 10 et 20. Ainsi ces rondelles 13 et 23 restent solidaires des vis 10 et 20 au cours de manipulations telles que montage ou stérilisation. A la figure 2, la rondelle 13 est représentée contre la tête de vis 12, pour permettre de voir le rétrécissement 16, tandis que la rondelle 23 est sensiblement dans la partie inférieure du rétrécissement 26.

Les vis 10 et 20 comportent encore une longueur filetée 17 et 27 respectivement, destinée à s'engager dans un taraudage correspondant, comme on le verra plus loin.

Les vis 10 et 20 et leurs rondelles 13 et 23 sont réalisées généralement en acier inoxydable, qui permet d'obtenir des pièces résistances et aptes à la stérilisation.

L'élément supérieur 30 comporte sur les axes 11 et 21 deux passages 31 et 32 pour les vis 10 et 20, dont la partie supérieure présente une creusure 33 et 34, destinée à coopérer avec la face sphérique 15 ou 25 des rondelles 13 et 23. Les creusures 33 et 34 sont soit sphériques, soit coniques. Pour éviter tout risque de rupture, il est nécessaire que les surfaces des parties sphériques 15 et 25 des rondelles, de même que celles des creusures 33 et 34, soient parfaitement polies.

La face inférieure de l'élément 30 comporte en outre une gorge 35, centrée entre les passages 31 et 32, comme visible à la figure 4. La gorge 35 est pourvue d'un évidement central 36 dont l'utilité sera expliquée plus loin.

L'élément de blocage 30 comporte encore des dégagements latéraux 37 et 38, disposés de part et d'autre de la gorge 35, et destinés à permettre une orientation angulaire des prolongements tubulaires 61 et 62 de la rotule 60 représentée à la figure 3.

L'élément inférieur 40 fait face à l'élément 30 décrit jusqu'ici. De manière analogue, il est pourvu de deux passages 41 et 42 sur les axes 11 et 21 des vis 10 et 20, d'une gorge 43 avec un évidement central 44, et de deux dégagements latéraux 45 et 46, tous ces éléments étant visibles soit en perspective à la figure 2, soit dans la vue latérale de la figure 3.

Dans sa partie inférieure, l'élément 40 comporte encore un prolongement d'appui 47, ainsi qu'une mâchoire 48 présentant une face inclinée 49, qui sont particulièrement visibles à la figure 3.

Le prolongement 47 sert de surface d'appui au côté 51 de la contre-pièce 50, dont les taraudages 52 et 53 sont prévus, sur les axes 11 et 21, pour recevoir les filetages 15 et 25 des vis 10 et 20.

Dans la forme d'exécution décrite ici, où le dispositif est prévu sur un arceau ayant une section 71 formée de deux triangles réunis par une âme (voir figure 1), la contre-pièce 50 est pourvue d'une mâchoire inclinée 54, destinée à la fixation de l'arceau 70 entre la mâchoire 48 de la pièce de blocage inférieure 40 et la mâchoire 54.

Comme on vient de le rappeler, la forme d'exécution décrite ici se rapporte à un dispositif prévu pour être monté sur un arceau. Pour cette raison, les éléments 30, 40 et 50 présentent une légère courbure, particulièrement visible à la figure 4, qui correspond à la courbure de l'arceau sur lequel est prévu le dispositif. Il va sans dire que dans d'autres formes d'exécution, les éléments de blocage 30 et 40, ainsi que la contre-pièce éventuelle 50, seront plats et non courbés.

De plus le dispositif de positionnement et de blocage représenté ici est prévu pour être serré sur la partie extérieure de l'arceau, mais sans sortir du cadre de la présente invention on pourrait bien sur réaliser également un dispositif destiné à être mis sur la partie intérieure de l'arceau.

Les éléments de blocage 30 et 40 ainsi que la contre-pièce 50 sont généralement réalisés en alliage léger, mais pourraient également être réalisés en une matière synthétique, apte à la stérilisation.

Quant à la rotule sphérique 63 destinée à être pincée entre les éléments 30 et 40, elle peut comporter deux prolongements tubulaires 61 et 62, comme représenté à la figure 3, disposés de part et d'autre de la sphère 63. Dans la variante des figures 1 et 2, elle ne comporte qu'un prolongement latéral 64, dont l'alésage central 65 sert de guidage à une tige d'armature ou autre pièce ou organe de liaison.

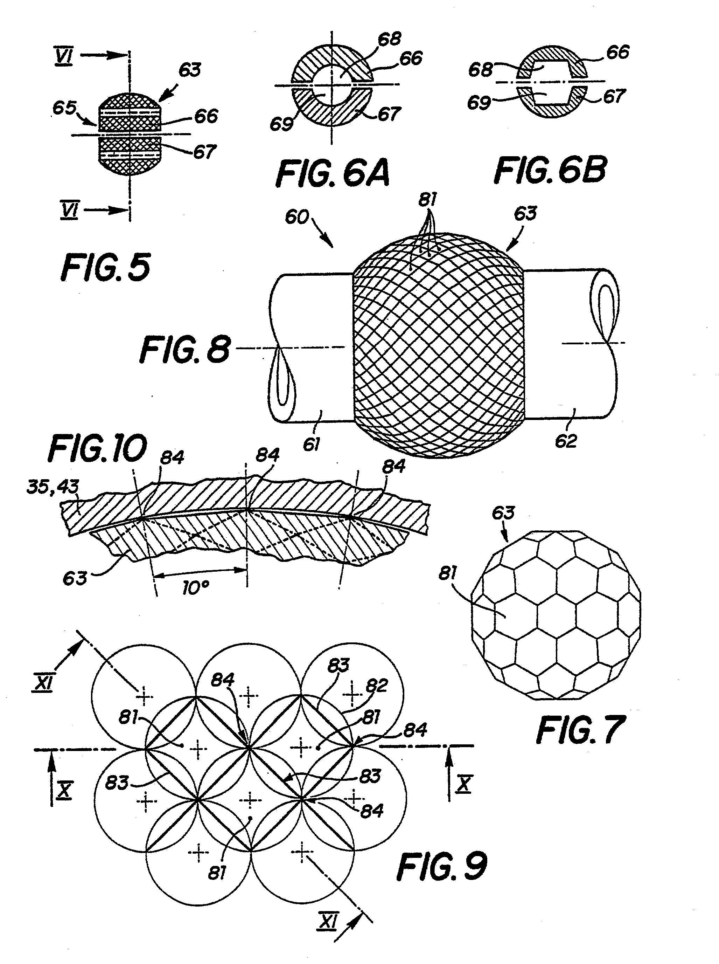

Dans la variante détaillée à la figure 5, la rotule est constituée uniquement par une partie sphérique 63. On remarque bien au dessin les petites facettes qui, bien qu'existantes, ne sont pas représentées aux figures 1 et 2 dans un but évident de clarification.

La rotule faisant l'objet des figures 5 et 6 est constituée par deux coquilles 66 et 67 qui comportent chacune un dégagement 68 ou 69. Ces dégagements sont dimensionnés pour permettre de serrer une tige de fixation ou une fiche transcutanée, disposée dans le passage central 65 et assurer son blocage dans le sens longitudinal et en rotation. Dans la variante de la figure 6A, le dégagement 68 ou 69 pratiqué dans chaque coquille 66 ou 67 a une section en forme de demi-cercle. En variante, le dégagement 69 a une section en forme de trapèze régulier, dont les côtés inclinés forment un angle supérieur ou égal à 7° par rapport à la perpendiculaire aux deux autres côtés, comme visible à la figure 6B. La sphère 63 de la rotule représentée à la figure 7 comporte des facettes hexagonales 81, mais il va sans dire que d'autres polygones sont également envisageables.

La figure 8 représente la partie centrale de la rotule 60 comportant des prolongements tubulaires 61 et 62 disposés de part et d'autre de la sphère représentée à la figure 3, cette partie centrale 63 ayant été considérablement agrandie. On remarque la succession de petites facettes 81, régulièrement espacées et disposées sur la surface de la rotule.

Un détail agrandi des facettes 81 est montré à la figure 9, indiquant que les facettes 81 sont inscrites dans des cercles adjacents 82, chaque groupe de cercle présentant en son centre un cercle supplémentaire de même diamètre, qui coupe les quatre autres cercles de manière à former des arêtes vives 83 ainsi que des coins 84 des facettes 81. Les cercles 82 sont dimensionnés de telle manière que les coins 84 soient inscrits dans un angle d'environ 10° par rapport au centre de la sphère 63.

La coupe de la figure 10, selon la ligne X-X de la figure 9, est réalisée de manière à couper tous les coins 84 d'une succession de facettes 81. Chaque coin 84 est donc une pointe qui viendra en contact avec les gorges 35 et 43 des éléments de blocage 30 et 40.

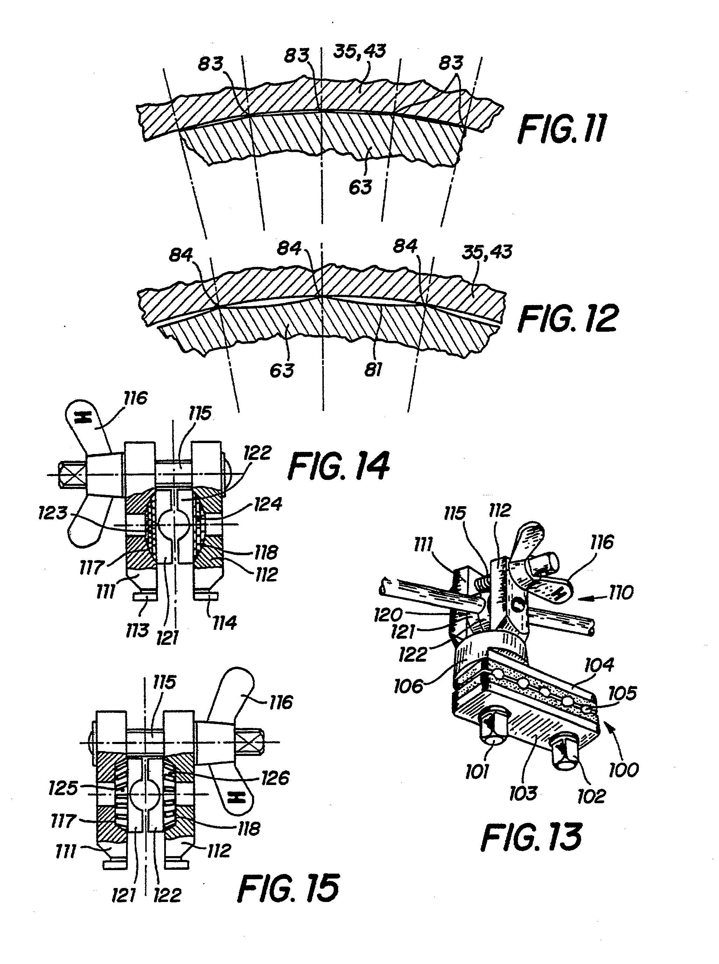

La coupe de la figure 11, selon XI-XI à la figure 9, est réalisée de manière à couper perpendiculairement les arêtes vives 83.

Alors que dans les figures 9 à 11, correspondant à un premier mode d'exécution, les facettes sont planes, dans la variante de la figure 12, qui correspond à la figure 11, les facettes 81 sont incurvées. On obtient ainsi des sommets 84 plus pointus et par conséquent plus efficaces pour le blocage dans les gorges 35 et 43 des mâchoires 30 et 40.

Il est d'autre part évident pour l'homme du métier que les facettes peuvent avoir d'autres formes, polygonales. Le but recherché par la présence des facettes, planes ou incurvées, peut être défini comme étant la possibilité de réaliser entre les dites facettes des arêtes vives ou des pointes qui permettront un accrochage efficace avec les mâchoires.

La pièce à rotule 60 est en général destinée à être traversée et à retenir des fiches ou tiges d'un diamètre de 2 à 8 mm. La partie sphérique 63 aura par conséquent habituellement un diamètre de 15 à 20 mm.

En règle générale, les mâchoires 30 et 40 seront réalisées dans un matériau d'une dureté plus faible que le matériau de la rotule 60. Ainsi l'enveloppe formée par les mâchoires sera donc plus élastique que la partie 63 de la rotule. En partant de ce fait, un grand nombre de matériaux peuvent être choisis. Par exemple, la rotule 60 peut être en acier trempé et les mâchoires en acier non trempé. On peut également associer une rotule en acier à des mâchoires en alliage léger, ou une rotule en alliage léger à des mâchoires présentant une dureté inférieure, par exemple en matière plastique.

L'utilisation du dispositif de positionnement et de blocage représenté aux figures 1 à 4 sera détaillée dès maintenant.

Selon chaque cas, on déterminera tout d'abord le genre de rotule 60 nécessitée dans le montage, soit une rotule avec deux prolongements latéraux 61 et 62 (voir figure 3), soit une rotule avec un prolongement 64 (figure 2), soit une rotule simplement sphérique, formée éventuellement de deux coquilles hémisphériques 66 et 67 (figure 5).

Le diamètre de l'alésage central 65 sera choisi en fonction des pièces à assembler.

On montera ensuite l'ensemble directement sur l'arceau, bien sûr sans bloquer encore les vis 10 et 20 dans les taraudages correspondants 52 et 53 de la pièce 50, et on disposera la pièce sphérique 60 ainsi que l'arceau 70 selon les nécessités.

On comprend dès lors l'intérêt des dégagements latéraux 37 et 38 de la pièce 30, ainsi que 45 et 46 de la pièce 40, qui permettent d'orienter les parties tubulaires 64 ou 61 et 62, sans être limité par les pièces 30 et 40 dans une plage d'orientation angulaire jusqu'à 40° environ.

Dès que le positionnement correct est atteint, on pourra serrer les vis 10 et 20, de manière à bloquer l'ensemble, qui pourra bien sûr toujours être ultérieurement desserré pour permettre tout réglage.

En se souvenant que les axes 11 et 21 ne sont pas rigoureusement parallèles, mais présentent un angle relatif d'environ 10°, on comprend dès lors l'intérêt de l'invention, qui permet d'obtenir un meilleur blocage, soit de la rotule, soit de l'arceau, puisque le serrage des vis peut fléchir les éléments de blocage 30 et 40.

La déformation due au fléchissement des pièces 30 et/ou 40 est compensée par le jeu des rondelles 13 et 23 dont la partie sphérique 15 ou 25 s'appuie différemment dans les creusures sphériques ou coniques 33 et 34. Ces rondelles permettent d'augmenter le serrage tout en diminuant le frottement. Pratiquement, on dimensionnera les parties sphériques 15 et 25 des rondelles pour que le contact avec la creusure correspondante se fasse environ au centre de la partie arrondie.

Il faut encore remarquer que les évidements 36 et 44 des éléments de blocage 30 et 40 respectivement ont pour rôle d'augmenter l'élasticité des pièces 30 et 40 et, par conséquent, de favoriser le blocage, puisqu'ils permettent de doubler le point d'appui sur la partie sphérique de la rotule.

Les figures 13 à 15 représentent un deuxième mode d'exécution d'un dispositif de positionnement et de serrage, également utilisé dans un fixateur externe. Ce dispositif est constitué principalement d'un ensemble de fixation 100 et d'un élément de positionnement et de blocage 110.

L'ensemble de fixation 100 est ici prévu pour être serré sur plusieurs fiches transfixiantes au moyen des vis 101 et 102, et à cet effet des mâchoires 103 et 104 comportent des alésages 105 destinés à serrer les fiches transfixiantes non représentées au dessin.

Cet ensemble de fixation 100 de fiches transfixiantes comporte en outre une partie cylindrique 106 présentant une rainure circulaire intérieure non représentée, destinée à coopérer avec l'élément de blocage 110.

A cet effet, l'élément 110 comporte deux pièces de blocage 111 et 112, dont la partie inférieure possède des pieds 113 et 114, destinés à s'engager dans la rainure circulaire intérieure du cylindre 106. Les pièces de blocage 111 et 112 sont traversées à leur extrémité supérieure par une tige filetée 115 serrée par un écrou 116. Les pièces 111 et 112 retiennent une rotule 120 constituée par deux pièces circulaires 121 et 122 (figure 14) qui elles-mêmes retiennent une tige dans un alésage commun aux deux pièces 121 et 122. Comme dans la version précédente, cet alésage peut présenter une section polygonale.

Lors du serrage des pièces 111, 112 sous l'action de l'écrou 116, les pieds 113, 114 s'appuieront fortement contre la rainure de la pièce 106 et empêcheront la rotation de l'ensemble supérieur 110. Les pièces de serrage 111, 112 comprennent des surfaces circulaires 117, 118, placées en regard l'une de l'autre, et dans lesquelles viendront s'appliquer des surfaces circulaires 123, 124 des pièces cylindriques 121, 122. Ces surfaces 123, 124 présentent des rangées de facettes rectangulaires formant entre elles des arêtes vives. Dans le mode d'exécution de la figure 14, les rangées de facettes sont disposées le long des segments ou zones sphériques divisant les calottes des parties latérales des pièces 121, 122. Les facettes sont ici des facettes planes réalisées dans un matériau plus dur que le matériau formant les pièces 111, 112.

Dans la variante de la figure 15, on retrouve les pièces 111, 112 présentant la même configuration que les pièces de la figure 14, les surfaces intérieures 117, 118 se présentant cependant sous forme d'un alésage en tronc de cône. Les pièces circulaires 121, 122 destinées à bloquer la tige présentent également des parties latérales en tronc de cône 125, 126, qui présentent une succession de facettes rectangulaires disposées selon les génératrices des parties en tronc de cône. Les facettes forment entre elles des arêtes vives qui viendront s'appuyer contre la partie en tronc de cône lisse correspondante des pièces 111, 112. Comme dans la variante ou dans les modes d'exécution précédents, les pièces 121, 122 seront réalisées dans un matériau plus dur que les pièces 111, 112.

Les modes d'exécution et variantes qui viennent d'être décrits proposent un dispositif de positionnement et de blocage d'une pièce à l'aide de mâchoires offrant une efficacité de blocage bien meilleure que tout ce qui est connu à ce jour dans l'état de la technique. Ce blocage efficace est obtenu en réalisant sur la pièce à bloquer une succession de facettes planes ou incurvées disposées sur des zones de la pièce à bloquer, de manière à ce que des facettes adjacentes forment entre elles des arêtes vives ou des pointes. La pièce à bloquer comprenant les facettes est réalisée dans un matériau plus dur que celui des mâchoires. Ceci permet aux arêtes vives ou aux pointes formées par les facettes d'obtenir des déformations élastiques dans les mâchoires, ce qui permet de réaliser un blocage particulièrement efficace. Il est évident pour l'homme de métier que des facettes pourraient être réalisées en variante sur les mâchoires et dans ce cas, ces mâchoires seront fabriquées dans un matériau plus dur que celui prévu pour la pièce à bloquer.

**Revendications**

1. Dispositif de positionnement et de blocage d'une pièce (60; 120), plus particulièrement utilisé dans le domaine des appareils de fixation externe d'os, comprenant deux mâchoires (30, 40; 111, 112) agencées pour être pressées l'une contre l'autre et enserrer la pièce, la pièce et/ou les mâchoires comprenant des zones circulaires présentant une succession de facettes régulièrement disposées sur les zones et destinées à coopérer avec la pièce ou la mâchoire complémentaire, caractérisé en ce que les facettes (81) sont constituées par des polygones réguliers adjacents sont planes ou incurvées et formant entre elles des arêtes vives ou des pointes et ou ce que lesdites facettes qui sont aptes à créer des déformations élastiques dans la pièce ou la mâchoire complémentaire, afin de réaliser un blocage efficace.

2. Dispositif selon la revendication 1, caractérisé par le fait que lesdites mâchoires (30, 40) sont serrées par au moins deux vis (10, 20) dont les axes (11, 21) forment un angle l'un par rapport à l'autre, l'une au moins desdites mâchoires étant apte à fléchir lors du serrage pour assurer un meilleur blocage de la pièce.

3. Dispositif selon la revendication 2, caractérisé en ce que les vis traversent librement lesdites mâchoires, leur tête (12, 22) appuyant au moins indirectement sur la mâchoire supérieure (30) et leur extrémité filetée (17, 27) coopérant avec un taraudage correspondant pratiqué dans une contre-pièce (40, 50).

4. Dispositif selon la revendication 3, caractérisé, en ce que la contre-pièce est confondue avec l'une desdites mâchoires.

5. Dispositif selon la revendication 3, caractérisé en ce que la tête de vis (12, 22) coopère avec une rondelle (13, 23) permettant de compenser le fléchissement de la mâchoire.

6. Dispositif selon la revendication 5, caractérisé en ce que la rondelle comporte une partie sphérique (15, 25).

7. Dispositif selon la revendication 6, caractérisé en ce que la mâchoire supérieure est munie d'un dégagement (33, 34) coopérant avec la partie sphérique de la rondelle.

8. Dispositif selon la revendication 7, caractérisé en ce que le dégagement est sphérique.

9. Dispositif selon la revendication 7, caractérisé en ce que le dégagement est conique.

10. Dispositif selon la revendication 5, caractérisé en ce que la rondelle (13, 23) est pincée dans un rétrécissement (16, 26) de la partie médiane de la vis, afin d'en rester solidaire.

11. Dispositif selon la revendication 2, caractérisé en ce que la pièce (60) à positionner et bloquer présente une partie arrondie (63) à enveloppe sphérique.

12. Dispositif selon la revendication 11, caractérisé en ce que la partie à enveloppe sphérique comporte un passage central (65).

13. Dispositif selon la revendication 11, caractérisé en ce que la pièce (60) est en acier inoxydable.

14. Dispositif selon la revendication 11, caractérisé en ce que la matière de la pièce (60) présente une dureté supérieure à celle des mâchoires (30, 40) l'entourant.

15. Dispositif selon la revendication 12, caractérisé en ce que la partie sphérique (63) est prolongée par au moins une partie tubulaire (61, 62, 64) s'étendant autour du passage central (65).

16. Dispositif selon la revendication 12, caractérisé en ce que la partie sphérique (63) est constituée par deux coquilles (66, 67) comportant un évidement latéral (68, 69) formant la moitié du passage central.

17. Dispositif selon la revendication 12, caractérisé en ce que le passage central (65) présente une section polygonale.

18. Dispositif selon la revendication 12, caractérisé en ce que le passage central (65) présente une section ellipsoïdale.

19. Dispositif selon la revendication 12, caractérisé en ce que le passage central (65) constitue un guide de fixation.

20. Dispositif selon la revendication 12, caractérisé en ce que le passage central correspond à une barre d'armature.

21. Dispositif selon la revendication 1, caractérisé en ce que les mâchoires (30, 40; 111, 112) entourant la pièce possèdent une creusure (35, 43; 117, 118) correspondant à la forme de la pièce.

22. Dispositif selon les revendications 2 et 21, caractérisé en ce que la creusure (35, 43) est centrée dans le plan passant par les vis de fixation.

23. Dispositif selon la revendication 22, caractérisé en ce que la creusure comporte au moins un évidement (36, 44) destiné à augmenter l'élasticité de la mâchoire.

24. Dispositif selon les revendications 15 et 21, caractérisé en ce que la creusure est bordée par deux dégagements (37, 38; 45, 46) situés de part et d'autre du plan passant par les vis de fixation, permettant le passage de la partie tubulaire (61, 62, 64).

25. Dispositif selon la revendication 3, caractérisé en ce que la mâchoire de blocage inférieure (40) et la contre-pièce (50) sont constituées de manière à former un étau, permettant leur fixation sur une armature (70) d'appareil de fixation squelettique.

26. Dispositif selon la revendication 25, caractérisé en ce que l'armature est constituée au moins partiellement par une partie d'arceau.

27. Dispositif selon la revendication 26, caractérisé en ce que les mâchoires dudit étau présentent une courbure correspondant à celle de l'arceau.

**Patentansprüche**

1. Einstell- und Blockierungsvorrichtung für ein Teil (60; 120), die ganz besonders auf dem Gebiet der Apparaturen zur externen Knochenfixierung Anwendung findet und aus zwei Backen (30, 40; 111, 112) besteht, die aneinandergedrückt werden und das Teil umgreifen können, wobei das Teil und/oder die Backen aus kreisförmigen Zonen mit einer Abfolge von Facetten bestehen, die regelmäßig auf den Zonen angeordnet und dazu bestimmt sind, mit dem Teil oder der ergänzenden Backe zusammenzuwirken, dadurch gekennzeichnet, daß die Facetten (81) aus regelmäßigen Polygonen aufgebaut sind und daß jene nebeneinanderliegenden Facetten entweder eben oder gekrümmt sind und zwischen sich scharfe Kanten oder Punkte bilden, die zur Herbeiführung einer wirksamen Blockierung elastische Verformungen in dem Teil oder der ergänzenden Backe erzeugen können.

2. Vorrichtung nach Anspruch 1, dadurch gekennzeichnet, daß jene Backen (30, 40) durch mindestens zwei Schrauben (10, 20) festgespannt sind, deren Achsen (11, 21) gegenseitig einen Winkel einschließen, wobei mindestens eine jener Backen dazu in der Lage ist, sich während des Spannens zu biegen, um eine bessere Blockierung des Teils zu gewährleisten.

3. Vorrichtung nach Anspruch 2, dadurch gekennzeichnet, daß die Schrauben frei durch die Backen hindurchgehen, während sich ihr Kopf (12, 22) mindestens indirekt auf der oberen Backe (30) abstützt und ihr Gewindeende (17, 27) mit einer entsprechenden, in einem Gegenstück (40, 50) ausgeführten Gewindebohrung zusammenwirkt.

4. Vorrichtung nach Anspruch 3, dadurch gekennzeichnet, daß das Gegenstück mit einer jener Backen vereinigt wird.

5. Vorrichtung nach Anspruch 3, dadurch gekennzeichnet, daß der Schraubenkopf (12, 22) mit einer Scheibe (13, 23) zusammenwirkt, wodurch das Biegen der Backe ausgeglichen werden kann.

6. Vorrichtung nach Anspruch 5, dadurch gekennzeichnet, daß die Scheibe aus einer kugelförmigen Sektion (15, 25) besteht.

7. Vorrichtung nach Anspruch 6, dadurch gekennzeichnet, daß die obere Backe mit einem Durchgang (33, 34) versehen ist, der mit der kugelförmigen Sektion der Scheibe zusammennwirkt.

8. Vorrichtung nach Anspruch 7, dadurch gekennzeichnet, daß der Durchgang kugelförmig ist.

9. Vorrichtung nach Anspruch 7, dadurch gekennzeichnet, daß der Durchgang konisch ist.

10. Vorrichtung nach Anspruch 5, dadurch gekennzeichnet, daß die Scheibe (13, 23) in einer Verengung (16, 26) der mittleren Sektion der Schraube verklemmt ist, um dort sicher zu sitzen.

11. Vorrichtung nach Anspruch 2, dadurch gekennzeichnet, daß das Teil (60), das eingestellt und blockiert werden soll, eine abgerundete Sektion (63) in Kugelform ist.

12. Vorrichtung nach Anspruch 11, dadurch gekennzeichnet, daß die Sektion in Kugelform einen Mittelgang (65) umfaßt.

13. Vorrichtung nach Anspruch 11, dadurch gekennzeichnet, daß das Teil (60) aus rostfreiem Stahl ist.

14. Vorrichtung nach Anspruch 11, dadurch gekennzeichnet, daß das Material des Teils (60) eine jenem der es umgebenden Backen (30, 40) überlegene Härte besitzt.

15. Vorrichtung nach Anspruch 12, dadurch gekennzeichnet, daß die kugelförmige Sektion (63) durch mindestens eine sich um den Mittelgang (65) erstreckende rohrförmige Sektion (61, 62, 64) verlängert ist.

16. Vorrichtung nach Anspruch 12, dadurch gekennzeichnet, daß die kugelförmige Sektion (63) aus zwei Schalen (66, 67) aufgebaut ist, die eine seitliche Aussparung (68, 69) umfassen, die die Hälfte des Mittelganges bildet.

17. Vorrichtung nach Anspruch 12, dadurch gekennzeichnet, daß der Mittelgang (65) einen polygonen Querschnitt aufweist.

18. Vorrichtung nach Anspruch 12, dadurch gekennzeichnet, daß der Mittelgang (65) einen elliptischen Querschnitt aufweist.

19. Vorrichtung nach Anspruch 12, dadurch gekennzeichnet, daß der Mittelgang (65) eine Fixierungsführung darstellt.

20. Vorrichtung nach Anspruch 12, dadurch gekennzeichnet, daß der Mittelgang einer Haltegerüststange entspricht.

21. Vorrichtung nach Anspruch 1, dadurch gekennzeichnet, daß die Backen (30, 40; 111, 112), die das Teil umgeben, eine Aushöhlung (35, 43; 117, 118) besitzen, die der Form des Teils entspricht.

22. Vorrichtung nach Ansprüchen 2 und 21, dadurch gekennzeichnet, daß die Aushöhlung (35, 43) in der Mitte der durch die Fixierungsschrauben gehenden Ebene liegt.

23. Vorrichtung nach Anspruch 22, dadurch gekennzeichnet, daß die Aushöhlung mindestens eine Aussparung (36, 44) umfaßt, die die Elastizität der Backe erhöhen soll.

24. Vorrichtung nach Ansprüchen 15 und 21, dadurch gekennzeichnet, daß die Aushöhlung von zwei Durchgängen (37, 38; 45, 46) begrenzt wird, die sich auf beiden Seiten der durch die Fixierungsschrauben gehenden Ebene befinden und das Hindurchgehen der rohrförmigen Sektion (61, 62, 64) ermöglichen.

25. Vorrichtung nach Anspruch 3, dadurch gekennzeichnet, daß die untere Blockierungsbacke (40) und das Gegenstück (50) so ausgebildet sind, daß sie einen Schraubstock bilden, wodurch sie auf einem Haltegerüst (70) der Skelettfixierungsapparatur fixiert werden können.

26. Vorrichtung nach Anspruch 25, dadurch gekennzeichnet, daß das Haltegerüst mindestens teilweise aus einer Bogensektion besteht.

27. Vorrichtung nach Anspruch 26, dadurch gekennzeichnet, daß die Backen dieses Schraubstockes eine Bogenlinie darstellen, die der des Bogens entspricht.

**Claims**

1. Positioning and immobilizing apparatus for a part (60; 120), more particularly used in the field of devices for external fixation of bones, comprising two jaws (30, 40; 111, 112) arranged so as to be pressed one against the other and to enclose the part, the part and/or the jaws comprising circular zones exhibiting a series of facets evenly arranged on the zones and intended to cooperate with the complementary part or jaw, characterized in that the facets (81) consist of regular polygons and in that the said adjacent facets are plain or incurved and form between them sharp edges or points which are capable of creating elastic deformations in the complementary part or jaw, in order to achieve an effective immobilization.

2. Apparatus according to Claim 1, characterized by the fact that the said jaws (30, 40) are tightened by at least two screws (10, 20) whose axes (11, 21) form an angle relative to one another, at least one of the said jaws being capable of bending during clamping in order to provide a better immobilization of the part.

3. Apparatus according to Claim 2, characterized in that the screws pass freely through the said jaws, their head (12, 22) bearing at least indirectly on the upper jaw (30) and their threaded end (17, 27) cooperating with a corresponding tapping made in a counter-part (40, 50).

4. Apparatus according to Claim 3, characterized in that the counter-part is identical to one of the said jaws.

5. Apparatus according to Claim 3, characterized in that the screw head (12, 22) cooperates with a washer (13, 23) permitting compensation of the bending of the jaw.

6. Apparatus according to Claim 5, characterized in that the washer comprises a spherical section (15, 25).

7. Apparatus according to Claim 6, characterized in that the upper jaw is provided with a clearance (33, 34) cooperating with the spherical section of the washer.

8. Apparatus according to Claim 7, characterized in that the clearance is spherical.

9. Apparatus according to Claim 7, characterized in that the clearance is conical.

10. Apparatus according to Claim 5, characterized in that the washer (13, 23) is gripped in a narrow section (16, 26) of the middle section of the screw, in order to remain integral therewith.

11. Apparatus according to Claim 2, characterized in that the part (60) to be positioned and immobilized has a rounded section (63) with a spherical shape.

12. Apparatus according to Claim 11 , characterized in that the section with a spherical shape comprises a central passage (65).

13. Apparatus according to Claim 11, characterized in that the part (60) is of stainless steel.

14. Apparatus according to Claim 11, characterized in that the material of the part (60) has a hardness greater than that of the jaws (30, 40) surrounding it.

15. Apparatus according to Claim 12, characterized in that the spherical section (63) is prolonged by at least one tubular section (61, 62, 64) extending around the central passage (65).

16. Apparatus according to Claim 12, characterized in that the spherical section (63) consists of two shells (66, 67) comprising a lateral recess (68, 69) forming half of the central passage.

17. Apparatus according to Claim 12, characterized in that the central passage (65) exhibits a polygonal cross-ection.

18. Apparatus according to Claim 12, characterized in that the central passage (65) exhibits an ellipsoid cross-section.

19. Apparatus according to Claim 12, characterized in that the central passage (65) constitutes a fixation guide.

20. Apparatus according to Claim 12, characterized in that the central passage corresponds to a frame bar.

21. Apparatus according to Claim 1, characterized in that the jaws (30, 40; 111, 112) surrounding the part exhibit a hollow (35, 43; 117, 118) corresponding to the shape of the part.

22. Apparatus according to Claims 2 and 21, characterized in that the hollow (35, 43) is centered in the plane passing via the fixation screws.

23. Apparatus according to Claim 22, characterized in that the hollow comprises at least one recess (36, 44) intended to increase the elasticity of the jaw.

24. Apparatus according to Claims 15 and 21, characterized in that the hollow is bordered by two clearances (37, 38; 45, 46) situated on either side of the plane passing via the fixation screws, permitting the passage of the tubular section (61, 62, 64).

25. Apparatus according to Claim 3, characterized in that the lower immobilizing jaw (40) and the counterpart (50) are made up in such a way as to form a vice, permitting their fixation on a frame (70) of a skeletal fixation device.

26. Apparatus according to Claim 25, characterized in that the frame consists at least partially of an arcuate section.

27. Apparatus according to Claim 26, characterized in that the jaws of the said vice exhibit a curvature corresponding to that of the arc.

FIG.1

FIG.2

FIG.3

FIG.4

EP 0 227 594 B1

FIG.5

FIG. 6A

FIG. 6B

FIG. 8

FIG.10

FIG. 7

FIG. 9

*FIG.11*

*FIG.12*

*FIG.14*

*FIG.13*

*FIG.15*